(19) **Europäisches Patentamt**
**European Patent Office**
**Office européen des brevets**

(11) **EP 1 289 495 B1**

(12) **EUROPEAN PATENT SPECIFICATION**

(45) Date of publication and mention
of the grant of the patent:
**29.09.2004 Bulletin 2004/40**

(51) Int Cl.[7]: **A61K 9/06**, A61K 31/522

(21) Application number: **01947701.7**

(86) International application number:
**PCT/HU2001/000069**

(22) Date of filing: **13.06.2001**

(87) International publication number:
**WO 2001/095878 (20.12.2001 Gazette 2001/51)**

(54) **PROCESS FOR THE PREPARATION OF A STERILE OINTMENT CONTAINING ACYCLOVIR**

VERFAHREN ZUR HERSTELLUNG VON STERILSALBE ENTHALTEND ACYCLOVIR

PROCEDE DE PREPARATION D'UN ONGUENT STERILE CONTENANT DE L'ACICLOVIR

(84) Designated Contracting States:
**AT BE CH CY DE DK ES FI FR GB GR IE IT LI LU MC NL PT SE TR**
Designated Extension States:
**AL LT LV MK RO SI**

(30) Priority: **14.06.2000 HU 0002266**

(43) Date of publication of application:
**12.03.2003 Bulletin 2003/11**

(73) Proprietor: **EGIS GYOGYSZERGYAR RT.**
**1106 Budapest (HU)**

(72) Inventors:
• **NAGY, Kálmánné**
**H-1025 Budapest (HU)**
• **BARANEK, Katalin**
**H-1204 Budapest (HU)**
• **SZELI, Jenoné**
**H-1162 Budapest (HU)**
• **EGRI, János**
**H-1036 Budapest (HU)**
• **MOSONYI, Antal;**
**H-9900 Körmend (HU)**
• **CLEMENTIS, Györgyné**
**H-1118 Budapest (HU)**

(74) Representative: **Beszédes, Stephan G., Dr. et al**
**Patentanwalt,**
**Münchener Strasse 80a**
**85221 Dachau (DE)**

(56) References cited:
**WO-A-00/01390**          DE-A- 19 705 277
**US-A- 5 300 296**

## Description

### Field of the invention

The invention refers to a process for the preparation of a sterile ointment, especially an eye ointment, containing acyclovir or a pharmaceutically suitable acid addition salt thereof as the active ingredient by homogenizing micronized acyclovir or a pharmaceutically acceptable acid addition salt thereof and the sterile carriers of the ointment in an aseptic way, then filling the homogenous ointment into sterile containers and sealing the containers.

### Background of the invention

From GB-P No. 1 523 865 it is known that acyclovir, chemically 9-[(2-hydroxyethoxy)methyl]guanine, possesses antiviral effect against various classes of DNA and RNA viruses. An emulsion of the type oil/water, essentially an aqueous cream, is described in EP No. 44,543 and the corresponding HU-P No. 185 936. The aqueous phase of the known emulsion contains the acyclovir or a pharmaceutically suitable acid addition salt thereof, while the oil phase contains oil, paraffin etc. The known composition can be used for the treatment of local viral infections on the skin surface, however, there is no hint of the preparation of a sterile composition or a sterile eye ointment.

DE 19705277 describes a sterile ointment comprising acyclovir made by sterile filtration of a solution of acyclovir in the sterile base of the ointment. There is no heating step required.

The aim of the invention is to provide a process for the preparation of a sterile ointment, especially an eye ointment, containing acyclovir or a pharmaceutically suitable acid addition salt thereof as the active ingredient, wherein said active ingredient is suspended in the carriers.

According to the quality requirements, the suspended active ingredient should be micronized, the size of the particles being suitably not higher than 60 μm. For the preparation of an eye ointment, the active ingredient employed should consist of even smaller particles. In our experience, an eye ointment corresponds to the the quality requirements of the various pharmacopeias, if, in any case, 100 % of the solid acyclovir particles are lower than 20 μm, and 90 % of the solid particles are lower than 10 μm in size.

While also non-sterile ointments are not allowed to contain pathogenic microorganisms, a certain germ number is allowable in accordance with the requirements for microbiological purity. In general, the total germ number should not be higher than 100 CFU (colony forming unit) in 1 g of ointment. However, in case of a sterile ointment, the total germ number must be equivalent to zero. In principle, such a microbiological purity can be obtained in either of two ways:

-    admixing a sterile active ingredient and sterile carriers under germ-free conditions; or
-    admixing non-sterile ingredients, then sterilizing the formed ointment by heat treatment.

The carriers can be sterilized by heat treatment without difficulties, however, no acyclovir active ingredient is commercially available which is sterile and consists of the small particles defined above.

In our experiments, it was tried to prepare sterile ointment by sterilizing a mixture of non-sterile acyclovir and non-sterile carriers. The non-sterile ointment was poured into a container, then sealed hermetically, and kept in a hot air sterilizing apparatus at 140 °C for 3 hours. Although, the total germ number was reduced to zero by the treatment, a great number of prism and needle shaped crystals and lumps of the size 200-300 μm could be observed in the ointment under microscope. This fact indicates unanimously that, at the high temperature employed, a part of the active ingredient dissolved in the carriers, then precipitated on cooling. In addition, even the consistence of the ointment did not correspond to the pharmacopeial requirements. A similar result was obtained when the ointment in the sealed container was sterilized under superpressure effected by saturated steam at 121 °C for 20 minutes. [These sterilization conditions are prescribed by the European Pharmacopeia, 3th edition, 1997.] Thus, subsequent sterilization of the mixed ointment could not produce the required result.

The next attempt consisted in the sterilization of the micronized acyclovir alone in a covered container that was not sealed hermetically. The sterilization treatment was performed under a superpressure effected by saturated steam at 121 °C for 20 minutes and in a hot air sterilizing apparatus at 140 °C for 3 hours, respectively. In further experiments, the active ingredient was subjected to a heat treatment at 160 °C for 45 minutes in a covered container that was not sealed hermetically. Although in each case sterile acyclovir was obtained, however, the particle sizes became so high that the particles were no longer suitable for the preparation of an eye ointment that corresponds to the pharmacopeial requirements.

In summary, none of the methods outlined above could produce a sterile eye ointment that corresponds to the pharmacopeial prescriptions.

Then it was tried to sterilize micronized acyclovir previously suspended in liquid paraffin or vaseline taken in a tenfold mass ratio relative to the amount of acyclovir. The suspensions were kept in containers that were covered but not sealed hermetically, and the heat treatments described above were employed. In each case a germ-free suspension was obtained, however, the acyclovir particles became so large that they were no more suitable for the preparation of an eye ointment that corresponds to the pharmacopeial prescriptions.

**Summary of the invention**

Surprisingly, it has been found that a sterile ointment, especially an eye ointment, that corresponds in every respect to the pharmacopeial requirements can be easily prepared by the process of the invention, in which 1 part by mass of acyclovir or a pharmaceutically suitable acid addition salt thereof consisting of particles that are lower in size than 60 μm, preferably 20 μm, are admixed to 1-30 parts by mass of vaseline and/or liquid paraffin and/or a mixture of solid and liquid paraffin, the mixture is hermetically sealed and kept at 120-140 °C for 0.3-5 hours, and the sterile mixture obtained is homogenized with a further sterile portion of the carriers in an aseptic way in a manner known *per se*.

For the expert it is surprising that the process of the invention provides a sterile acyclovir suspension containing acyclovir particles of practically unchanged sizes, thus, being suitable for the preparation of a sterile eye ointment that corresponds to the pharmacopeial requirements, since, during various sterilizing heat treatments discussed above in detail, the particles of acyclovir increased significantly and were no longer suitable for the preparation of an eye ointment. It was not to be expected that the acyclovir particles remained of unchanged size when subjected to heat treatment in a suspension sealed hermetically.

The results of our sterilizing experiments are summarized in Tables I and II. In each case the particle size of the acyclovir used was the following: 100 % of the particles were lower than 20 μm, and 90 % of the particles were lower than 10 μm. When a suspension of acyclovir was subjected to the heat treatment, always 1 part by mass of acyclovir was admixed to 10 parts by mass of carrier that was either liquid paraffin or vaselin. Three kinds of heat treatment were employed:

- treatment at 121 °C in an autoclave under a superpressure effected by saturated steam at 121 °C for 20 minutes;
- treatment in a hot air sterilizing apparatus at 140 °C for 3 hours;
- treatment in a hot air sterilizing apparatus at 160°C for 45 minutes.

In the experiments summarized in Table I, the test samples were kept in containers which were not sealed hermetically, only covered by a sterilization foil. In case of the experiments summarized in Table II, the test samples were kept in sterilizing bottles sealed hermetically.

Table I

| Heat sterilization of acyclovir particles using samples placed in covered but hermetically not sealed containers | | |
|---|---|---|
| **Treatment mode** | **Sterility** | **Acyclovir particle size in** μ**m** |
| acyclovir kept at 121 °C for 20 minutes | sterile | 20-350 |
| acyclovir kept at 140 °C for 3 hours | sterile | 30-300 |
| acyclovir kept at 160 °C for 45 minutes | sterile | 60-320 |
| **control:** acyclovir suspended in liquid paraffin, without heat treatment | non-sterile | lower than 20 |
| acyclovir suspended in liquid paraffin, 121 °C, 20 minutes | sterile | 30-100 |
| acyclovir suspended in liquid paraffin, 140 °C, 3 h | sterile | 30-140 |
| acyclovir suspended in liquid paraffin, 160 °C, 45 minutes | sterile | 35-180 |
| **control:** acyclovir suspended in vaseline, without heat treatment | non-sterile | lower than 20 |
| acyclovir suspended in vaseline, 121 °C, 20 minutes | sterile | 40-100 |
| acyclovir suspended in vaseline, 140 °C, 3h | sterile | 50-130 |
| acyclovir suspended in vaseline, 160 °C, 45 minutes | sterile | 40-180 |

It can be seen from Table I that the double requirements relating to sterility and particle size could not be fulfilled in the cases examined.

Table 2

| Heat sterilization of acyclovir particles using samples placed in hermetically sealed containers | | |
|---|---|---|
| **Treatment mode** | **Sterility** | **Acyclovir particle size in** $\mu$**m** |
| acyclovir kept at 121 °C for 20 minutes | sterile | 20-180 |
| acyclovir kept at 140 °C for 3 hours | sterile | 30-200 |
| acyclovir kept at 160 °C for 45 minutes | sterile | 60-400 |
| **control:** acyclovir suspended in liquid paraffin, without heat treatment | non-sterile | lower than 20 |
| acyclovir suspended in liquid paraffin, 121 °C, 20 minutes | sterile | lower than 20 |
| acyclovir suspended in liquid paraffin, 140 °C, 3 h | sterile | lower than 20 |
| acyclovir suspended in liquid paraffin, 160 °C, 45 minutes | sterile | 10-50 |
| **control:** acyclovir suspended in vaseline, without heat treatment | non-sterile | lower than 20 |
| acyclovir suspended in vaseline, 121 °C, 20 minutes | sterile | lower than 20 |
| acyclovir suspended in vaseline, 140 °C, 3h | sterile | lower than 20 |
| acyclovir suspended in vaseline, 160 °C, 45 minutes | sterile | 10-60 |

The data of Table 11 indicate that the double requirements mentioned above could be fulfilled only by performing the heat treatment of the acyclovir particles in liquid paraffin or vaseline at 121 or 140°C in a closed space i. e. in a hermetically sealed container. When the heat treatment was carried out at 160 °C, an increase of the particles to a certain degree was experienced, thus, the suspension examined became unsuitable for the preparation of an eye ointment that corresponded to the pharmacopeial prescriptions.

**Description of the preferred embodiments**

Thus, according to the invention, a sterile ointment, especially an eye ointment, containing acyclovir or a pharmaceutically suitable acid addition salt thereof as the active ingredient is prepared by homogenizing micronized acyclovir or a pharmaceutically acceptable acid addition salt thereof and the sterile carriers of the ointment in an aseptic way, then filling the homogenous ointment into sterile containers and sealing the containers, in which 1 part by mass of acyclovir or a pharmaceutically suitable acid addition salt thereof consisting of particles that are lower in size than 60 $\mu$m, preferably 20 $\mu$m, are admixed to 1-30 parts by mass of vaseline and/or liquid paraffin and/or a mixture of solid and liquid paraffin, the mixture is hermetically sealed and kept at 120-140 °C for 0.3-5 hours, and the sterile mixture obtained is homogenized with a further sterile portion of the carriers in an aseptic way in a manner known *per se*.

The vaseline, liquid paraffin and solid paraffin in which the particles of the active ingredient are suspended and treated with heat to obtain a sterile mixture are produced in the distillation of mineral oil. All these products are commercially available. The vaseline can be white vaseline (vaselinum album) or yellow vaseline (vaselinum flavum).

One or more conventional carrier(s) employed in ointments, especially eye ointments, can be used in the sterile ointment, for example, white vaseline, yellow vaseline, liquid paraffin, solid paraffin, lanolin, cholesterol, higher alkanols such as myristyl alcohol, cetyl alcohol, cetyl stearyl alcohol, glycerol, mono-, di- and triesters of the latter formed with fatty acids, preferably glycerol mono-stearate, propylene glycol, poly(ethylene glycol), fatty acid esters of poly(ethylene glycol), sorbitan fatty acid esters, water etc.

In the process of the invention, preferred carrier(s) are, in addition to the vaseline and/or liquid paraffin and/or a mixture of solid and liquid paraffin, as follows: lanolin, glycerol monostearate and cetyl alcohol.

The further sterile portion of the carriers may contain also one or more conventional microbiological preservative(s), preferably 2-phenylethanol in an amount of 0.3-5.0 % by mass, chlorobutanol in an amount of 0.3-0.6 % by mass or benzyl alcohol in an amount of 0.5-1.0 % by mass, related to the total amount of the ointment.

The vaseline, liquid paraffin and solid paraffin are suitably used in a ratio to obtain the desired consistence of the ointment.

Preferably, an oily ointment without water or an ointment of the type water/oil is prepared by the process of the invention, wherein the amount of the water is four times the mass of the active ingredient, at the most.

According to a preferred process of the invention, 1 part by mass of acyclovir or a pharmaceutically suitable acid addition salt thereof consisting of particles that are lower in size than 40 $\mu$m are admixed to 1-30 parts by mass of vaseline and/or liquid paraffin and/or a mixture of solid and liquid paraffin, the mixture is hermetically sealed and kept at 120-140 °C for 0.3-5 hours, and the sterile mixture obtained is homogenized with further 1-30 parts by mass of vaseline and/or liquid paraffin and/or a mixture of solid and liquid paraffin, 1-2 parts by mass of sterile lanolin, 0.5-1 parts by mass of a sterile glycerol ester, 0.5-1 parts by mass of a $C_{16-18}$ sterile alkanol and, if desired, 1-4 parts by mass of sterile water and/or 0.1-0.4 parts by mass of a sterile preservative.

According to an especially preferred process of the invention, 1 part by mass of the active ingredient is admixed to 1-10 parts by mass of vaseline or liquid paraffin, and the mixture obtained is subjected to the heat treatment.

Alternatively, the further non-sterile carriers used for the preparation of the ointment can be mixed with each other, sterilized, then homogenized with the sterilized suspension of acyclovir or a pharmaceutically suitable acid addition salt thereof in vaseline and/or liquid paraffin and/or a mixture of solid and liquid paraffin.

For the preparation of eye ointment, suitably an acyclovir or pharmaceutically suitable acid addition salt is employed which consists of particles that are lower in size than 20 μm, and 90 % of the particles are lower than 10 μm.

The process of the invention can be carried out in a very simple way and is suitable for the preparation of a sterile ointment, especially a sterile eye ointment.

The invention is further elucidated by means of the following

Examples.

Example 1

Preparation of an oily eye ointment

600 g of white vaseline is heated to about 60 °C, and, to the melt obtained, 60 g of micronized acyclovir consisting of particles that are lower in size than 20 μm, and 90 % by mass of the particles are lower than 10 μm are added. After thorough mixing, the suspension is filled into a sterilizing bottle of 1 litre capacity and the bottle is sealed with a cap. 1180 g of white vaseline, 100 g of lanolin, 30 g of glycerol monostearate and 30 g of cetyl alcohol are transfered into a sterilizing bottle of 2 litres capacity, and the bottle is sealed with a cap. The bottles are placed into a hot air sterilizing apparatus and sterilized at 140 °C for 3 hours. After the end of the sterilization, both the sterile suspension and the sterile mixture of the further carriers are allowed to cool to about 70 °C, then the contents of both sterilizing bottles are filled into a sterile apparatus mounted with a mixer, mixed at 70 °C for 10 minutes, cooled to 25 °C under constant stirring, the finished ointment is filled into sterile tubes that are sealed. Each tube contains 4.5 g of ointment.

The germ number of the ointment obtained was equivalent to zero and did not contain acyclovir particles higher in size than 20 μm. The eye ointment corresponded to the requirements of the European Pharmacopeia, 3rd edition, 1997 in every respect.

Both single acute and 28 days' chronic eye irritation tests were carried out on New-Zealand rabbits by administering doses of 100 mg of ointment into the right eye. Neither the single treatment, nor the chronic treatment repeated daily for 28 days caused any irritation in the eye of the rabbits.

Example 2

Preparation of an oily eye ointment

The procedure described in Example 1 is repeated with the difference that an eye ointment of the following composition is prepared:

| | |
|---|---|
| acyclovir (micronized) | 0.1350 g |
| vaseline, white | 3.9825 g |
| lanolin | 0.2250 g |
| glycerol monostearate | 0.0675 g |
| cetyl alcohol | 0.0675 g |
| 2-phenylethanol | 0.0225 g |
| | 4. 5000 g |

Example 3

Preparation of an oily eye ointment

The procedure described in Example 1 is repeated with the difference that an eye ointment of the following composition is prepared:

| acyclovir (micronized) | 0.2700 g |
|---|---|
| paraffin, liquid | 2.5650 g |
| vaseline, white | 1.2550 g |
| lanolin | 0.1400 g |
| glycerol monostearate | 0.1350 g |
| cetyl alcohol | 0.1350 g |
| | 4.5000 g |

Example 4

Preparation of an oily eye ointment

The procedure described in Example 1 is repeated with the difference that an eye ointment of the following composition is prepared:

| acyclovir (micronized) | 0.1350 g |
|---|---|
| paraffin, liquid | 2.7000 g |
| paraffin, solid | 1.3050 g |
| lanolin | 0.2250 g |
| glycerol monostearate | 0.0675 g |
| cetyl stearyl alcohol | 0.0675 g |
| | 4.5000 g |

Example 5

Preparation of an eye ointment containing water (of the type water/oil)

The procedure described in Example 1 is repeated with the difference that an eye ointment of the following composition is prepared:

| acyclovir (micronized) | 0.1350 g |
|---|---|
| paraffin, liquid | 0.9675 g |
| vaseline, white | 2.7000 g |
| lanolin | 0.2025 g |
| 2-phenylethanol | 0.0450 g |
| distilled water for injections | 0.4500 g |
| | 4.5000 g |

**Claims**

1.  A process for the preparation of a sterile ointment, especially an eye ointment, containing acyclovir or a pharmaceutically suitable acid addltion salt thereof as the active ingredient by homogenizing micronized acyclovir or a pharmaceutically acceptable acid addition salt thereof and the sterile carriers of the ointment in an aseptic way, then filling the homogenous ointment into sterile containers and sealing the containers, **in which**
    1 part by mass of acyclovir or a pharmaceutically suitable acid addition salt thereof consisting of particles that are lower in size than 60 µm, preferably 20 µm, are admixed to 1-30 parts by mass of vaseline and/or liquid paraffin and/or a mixture of solid and liquid paraffin, the mixture is hermetically sealed and kept at 120-140 °C for 0.3-5 hours, and the sterile mixture obtained is homogenized with a further sterile portion of the carriers in an aseptic way in a manner known *per* se.

2. A process as claimed in Claim 1, **in which**
the sterile mixture of acyclovir and vaseline and/or liquid paraffin and/or a mixture of solid and liquid paraffin is homogenized with further 1-30 parts by mass of sterile vaseline and/or liquid paraffin and/or a mixture of solid and liquid paraffin, 1-2 parts by mass of sterile lanolin, 0.5-1 parts by mass of a sterile glycerol ester, 0.5-1 parts by mass of a $C_{16-18}$ sterile alkanol and, if desired, 1-4 parts by mass of sterile water and/or 0.1-0.4 parts by mass of a sterile preservative.

3. A process as claimed in Claim 1 or 2 for the preparation of an eye ointment, **in which,**
the particles of acyclovir or a pharmaceutically suitable acid addition salt thereof are lower in size than 20 μm.

**Patentansprüche**

1. Verfahren zur Herstellung einer sterilen Salbe, insbesondere einer Augensalbe, enthaltend Acyclovir oder ein pharmazeutisch geeignetes Säureadditionssalz davon als aktiven Bestandteil, durch Homogenisieren von mikronisiertem Acyclovir oder einem pharmazeutisch annehmbaren Säureadditionssalz davon und den sterilen Trägem der Salbe in aseptischer Weise, anschließendes Füllen der homogenen Salbe in sterile Behälter und Verschließen der Behälter, wobei
1 Massenteil an Acyclovir oder eines pharmazeutisch geeigneten Säureadditionssalzes davon, bestehend aus Teilchen, die eine geringere Größe als 60 μm, vorzugsweise 20 μm, besitzen, zu 1-30 Massenteilen Vaseline und/ oder flüssigem Paraffin und/oder einer Mischung von festem und flüssigem Paraffin hinzugemischt wird, die Mischung hermetisch verschlossen wird und bei 120-140 °C 0,3-5 Stunden lang gehalten wird und die erhaltene sterile Mischung mit einem weiteren sterilen Teil der Träger in aseptischer Weise in einer *per se* bekannten Weise homogenisiert wird.

2. Verfahren gemäß Anspruch 1, bei dem die sterile Mischung aus Acyclovir und Vaseline und/oder flüssigem Paraffin und/oder einer Mischung aus festem und flüssigem Paraffin mit weiteren 1-30 Massenteilen an steriler Vaseline und/oder flüssigem Paraffin und/oder einer Mischung von festem und flüssigem Paraffin, 1-2 Massenteilen sterilem Lanolin, 0,5-1 Massenteil eines sterilen Glycerolesters, 0,5-1 Massenteil eines sterilen $C_{15-18}$-Alkanols und, sofern erwünscht, 1-4 Massenteilen an sterilem Wasser und/oder 0,1-0,4 Massenteilen eines sterilen Konservierungsstoffes homogenisiert wird.

3. Verfahren gemäß Anspruch 1 oder 2 zur Herstellung einer Augensalbe, bei dem
die Teilchen aus Acyclovir oder einem pharmazeutisch geeigneten Säureadditionssalz davon eine geringere Größe als 20 μm besitzen.

**Revendications**

1. Procédé de fabrication d'un onguent stérile, en particulier un onguent ophtalmique, contenant comme ingrédient actif de l'aciclovir ou un sel d'addition d'acide de celui-ci convenable sur le plan pharmaceutique, par homogénéisation, dans des conditions d'asepsie, de l'aciclovir micronisé ou d'un sel d'addition d'acide de celui-ci acceptable sur le plan pharmaceutique micronisé, et des supports stériles de l'onguent, puis par remplissage de récipients stériles avec l'onguent homogénéisé et, enfin, par fermeture des récipients, dans lequel
1 partie en poids d'aciclovir ou d'un sel d'addition d'acide de celui-ci convenable sur le plan pharmaceutique, constituée de particules d'une taille inférieure à 60 μm, de préférence 20 μm, est mélangée à 1 à 30 parties en poids de vaseline et/ou de paraffine liquide et/ou d'un mélange de paraffine solide et liquide, le mélange étant hermétiquement fermé et conservé à 120 à 140°C pendant 0,3 à 5 heures, et le mélange stérile obtenu étant homogénéisé, dans des conditions d'asepsie, avec une fraction stérile supplémentaire des supports, d'une manière connue en soi.

2. Procédé selon la revendication 1, dans lequel le mélange stérile d'aciclovir et de vaseline et/ou de paraffine liquide et/ou d'un mélange de paraffine solide et liquide est homogénéisé avec 1 à 30 parties supplémentaire(s) en poids de vaseline et/ou de paraffine liquide et/ou d'un mélange de paraffine solide et liquide stériles, 1 à 2 parties en poids de lanoline stérile, 0,5 à 1 partie en poids d'un ester de glycérol stérile, 0,5 à 1 partie en poids d'un alcanol

C$_{16-18}$ stérile et, éventuellement, 1 à 4 parties en poids d'eau stérile et/ou 0,1 à 0,4 partie en poids d'un conservateur stérile.

3. Procédé selon les revendications 1 ou 2 pour la fabrication d'un onguent ophtalmique, dans lequel les particules d'aciclovir ou d'un sel d'addition d'acide de celui-ci convenable sur le plan pharmaceutique, présentent une taille inférieure à 20 $\mu$m.